# EUROPEAN PATENT SPECIFICATION

(11) **EP 2 996 580 B1**
(45) Date of publication and mention of the grant of the patent: **12.02.2020**
(21) Application number: 14782510.3
(22) Date of filing: 11.04.2014
(51) Int. Cl.: A61B 17/12, A61B 17/03, A61B 90/00, A61M 25/01, A61M 25/098, A61L 31/02, A61L 31/08, A61L 31/18, A61L 31/14

(54) **RADIOPAQUE DEVICES FOR CEREBRAL ANEURYSM REPAIR**
RÖNTGENDICHTE VORRICHTUNGEN FÜR REPARATUR VON ZEREBRALEM ANEURYSMA
DISPOSITIFS RADIO-OPAQUES POUR RÉPARATION D'UN ANÉVRISME CÉRÉBRAL

(30) Priority: 11.04.2013 US 201361811055 P; 27.12.2013 US 201361921338 P
(43) Date of publication of application: 23.03.2016
(73) Proprietor: BALT USA LLC, Irvine, CA 92618 (US)
(72) Inventor: FERRERA, David, Coto De Caza, CA 92679 (US); TAKAHASHI, Randall, Irvine, CA 92618 (US); LE, Dawson, Garden Grove, CA 92843 (US)
(74) Representative: Crease, Devanand John
(86) International application number: PCT/US2014/033881
(87) International publication number: WO 2014/169261

(56) References cited:
- WO-A2-2005/094486
- US-A1- 2006 127 443
- US-A1- 2007 036 905
- US-B1- 6 458 127
- US-B2- 7 208 172
- US-B2- 8 152 839
- US-B2- 8 152 839

## Description

### Cross-Reference to Related Application

This application claims the full Paris Convention priority of, and expressly incorporates by this reference U.S. provisional patent application 61/811,055, filed on April 11, 2013, as if fully set forth herein. Likewise, priority is claimed to PCT/US2014/031666; and U.S. provisional patent application 61/921,338, filed December 27, 2013.

### Field of Inventions

The field of the invention is compositions, devices, and methods to make devices to repair cerebral aneurysms, most particularly neurovascular treatment of cerebral aneurysms.

### Background

Cerebral aneurysms occur in approximately 2% of the population. Approximately 30,000 aneurysms are treated annually in the USA. Within this therapy group, 23,000 aneurysms are embolized with coils while 7,000 are repaired endoluminally with flow diverting devices. Aneurysms grow from a weakness in a blood vessel. About 80% of aneurysms are less than 8mm with the remainder growing to as large as 40mm. During stent-assisted coiling, a stent (Boston Scientific's Neuroform® brand of device or J&J Cordis Enterprise® brand of device) structure is placed within the artery of the vessel with the aneurysm in an attempt to reconstruct the vessel wall at the neck of the aneurysm. US 8152839 B2 for example relates to an embolic coil formed of a wound ribbon having a primary shape and a secondary shape, wherein: the primary shape is a straight shape; the secondary shape is selected from the group consisting of a spiral shape, a single apex vortex shape, a J shape, a helical shape, a complex helical shape and a straight shape; the wound ribbon is configured to exhibit its primary shape when disposed in a lumen of a medical device; the wound ribbon is configured to exhibit its secondary shape upon exit from the medical device; in its primary shape, the wound ribbon comprises consecutive windings of the ribbon that contact each other and are flush with each other; and the ribbon is a strip of material having a polygonal transverse cross-section.

However, patients with sub-arachnoid hemorrhage (SAH) are typically not candidates for stents due the prophylactic drug regimen to mitigate the thrombo-embolic complications. A second approach is to perform balloon-remodeling. In this technique, a very soft, conformable balloon (for example, the eV3 Hyperform® brand of device) typically is used for balloon-test-occlusion being placed in the artery at the neck to reconstruct the neck at the aneurysm origin.

However, during this technique, flow arrest is performed while the balloon is inflated. There is a risk of initiating an ischemic event during balloon remodeling and/or a thrombo-embolic event during flow arrest. Once both these techniques are performed, coil embolization of the aneurysm can be performed. During the stenting procedure, the stent may be permanently implanted. During balloon remodeling, the balloon is removed once embolization is completed. Coils thus remain state-of-the-art treatment in most of the world, and are important in that regard.

In addition, devices discussed so far, are typically visualized by markers at both the distal and proximal ends only. Although these markers verify the placement and/or deployment of the devices within the artery in relation to the abnormality to be repaired, verification of the complete opening or apposition to the artery wall or aneurysm neck cannot be verified unless a contract enhanced CT is performed. The ability to visualize these devices implanted in the artery offers procedural and clinical safety and benefit. The instant teachings address this need. Likewise, MRI-safe, namely, artifact-free imaging is a longstanding need in the field.

In most cases, complete embolization of the aneurysm with currently known devices will typically require at least several coils and devices, and/or are incomplete treatment of the aneurysm.

Further, it is not uncommon that sufficient amounts of devices cannot be placed into the aneurysm without losing microcatheter access due to aneurysm, aneurysm neck or vesicle morphology. Additionally, currently known devices can often not readily be stabilized within the aneurysm due to their visibility, softness and inertness. Finally, electrolytic detachment needs to be managed efficiently with proper timing for embolic coil delivery.

Many methods are described in the prior art for rendering stents or portions of stents radiopaque. WO 2005/094486 A2 for example relates to a medical device having a porous radiopaque coating that can withstand the high strains inherent in the use of such devices without delamination. A coating of Ta is applied to a medical device, such as a stent, by vapor deposition so that the thermomechanical properties of the stent are not adversely affected. The coating preferable has high emissivity. Other examples include filling cavities on the stent with radiopaque material (U.S. Pat. Nos. 6,635,082; 6,641,607), radiopaque markers attached to the stent (U.S. Pat. Nos. 6,293,966; 6,312,456; 6,334,871; 6,361,557; 6,402,777; 6,497,671; 6,503,271;
6,554,854), stents comprised of multiple layers of materials with different radiopacities (U.S. Pat. Nos. 6,638,301; 6,620,192), stents that incorporate radiopaque structural elements (U.S. Pat. Nos. 6,464,723; 6,471,721; 6,540,774; 6,585,757; 6,652,579),
coatings of radiopaque particles in binders (U.S. Pat. No. 6,355,058), and methods for spray coating radiopaque material on stents (U.S. Pat. No. 6,616,765).

All of the prior art methods for imparting radiopacity to stents significantly increase the manufacturing cost and complexity and/or render only a small part of the stents radiopaque. The most efficient method would be to simply apply a conformal coating of a fully dense radiopaque material to all surfaces of the stent. The coating would have to be thick enough to provide good X-ray contrast, biomedically compatible and corrosion resistant.

Thus, there is still a need for improved devices and methods of embolization devices, and especially neurovascular embolization devices for cerebral aneurysms, which are electrolytically detachable, sizable and present no artifacts based on materials involved using MRI.

### SUMMARY OF THE DISCLOSURE

In a first aspect, there is provided an intraluminal medical device system for placement within a neurovascular structure, which comprises, in combination: at least a flexible body; and a porous columnar metallic coating comprising a plurality of independent columnar structures separated from one another by an opening and disposed on at least a portion of the flexible body, wherein the porous columnar metallic coating extends in a substantially normal direction from a surface of at least a portion of the flexible body; flaking of the porous columnar metallic coating being less than 1% of the porous columnar metallic coated surface when the flexible body and porous columnar metallic coating are strained up to 8%; and
the combination of the size of the columnar structures of the porous columnar metallic coating and the size of the openings between the columnar structures of the porous columnar metallic coating results in an emissivity in the visible spectrum range of at least 80%;
wherein the flexible body partially comprises a radio transparent material and the porous columnar metallic coating on at least a portion of the flexible body comprises tantalum; wherein the porous columnar tantalum coating thickness is between approximately 3 and 10 microns;
wherein the porous columnar tantalum coating is applied to the flexible body by a physical vapor deposition process;
wherein the physical vapor deposition process includes one of the group of sputtering, cathodic arc deposition or thermal evaporation; and
the intraluminal medical device is an embolic coil;
further comprising a material in the porous columnar tantalum coating, wherein the material diffuses out over time.

In embodiments, there is provided an intraluminal medical device comprising flexible body which is further comprised of: an elastic shape retaining body having an outer surface, namely an embolic coil; the porous columnar metallic coating is disposed on at least a portion of the outer surface;wherein the porous columnar metallic coating is applied using a physical vapor deposition process.

In a further embodiment, there is provided an intraluminal medical device comprising a flexible body which is further comprised of an elastic shape retaining body at least partially comprising a material having superelasticity and shape memory.

In another embodiment, there is provided an intraluminal medical device comprising a porous columnar metallic coating which is a radiopaque coating.

In embodiments, there is provided an intraluminal medical device comprising a flexible body which is comprised of at least one of nitinol, and other high performance alloys, including 35N LT.

In further embodiments, there is provided an intraluminal medical device comprising a flexible body which is comprised of 35N LT.

In another embodiment, there is provided an intraluminal medical, wherein the flexible body is fabricated from a shape memory alloy having an A_{f} value below body temperature.

In embodiments, there is provided an intraluminal medical device, wherein the medical device diameter is between 2.0 and 5.0 mm.

In a further embodiment, there is provided an intraluminal medical device, wherein the medical device length is between 1 and 5 cm.

In another embodiment, there is provided an intraluminal medical device, wherein the medical device thickness is between 30 and 100 microns.

In a further aspect, there is provided a process for depositing a tantalum layer on a medical device, the method comprising the steps of:
maintaining a background pressure of inert gas in a sputter coating system containing a tantalum sputter target;
applying a voltage to the tantalum target to cause sputtering; and
sputtering for a period of time to produce the desired coating thickness;
wherein the tantalum layer preferably has an emissivity in the visible spectrum of at least 80%.

The present invention is directed towards a medical device having a radiopaque outer coating that is able to withstand the strains produced in the use of the device without delamination, in particular, embolic coils. Likewise, using 35N LT (high performance alloy - not stainless steel) in detachable delivery systems eliminates MRI artifacts from other materials. Times ranging between 5 and 14 seconds of detachment time are currently operating for systems as discussed herein. (Blockade Medical®'s Barricade® brand of coil, Irvine, California92618).

A process for depositing a Ta layer on a medical device consisting of the steps of: maintaining a background pressure of inert gas in a sputter coating system containing a Ta sputter target; applying a voltage to the Ta target to cause sputtering; and sputtering for a period of time to produce the desired coating thickness; wherein the Ta layer preferably has an emissivity in the visible spectrum of at least 80%. The device preferably is not directly heated or cooled and the equilibrium temperature of the device during deposition is controlled indirectly by the process. The equilibrium temperature preferably is between 150° and 450° C. A voltage, ac or de, can be applied steadily or in pulses to the medical device during the process. An initial high voltage, preferably between 300 and 500 volts, can be applied to preclean the device for a first period of time, preferably between 1 minute and 20 minutes. A second, lower voltage, preferably between 50 and 200 volts, can be applied for a period of time, preferably between 1 and 3 hours. Preferably, the inert gas is from the group comprising Ar, Kr and Xe. Preferably, the voltage on the target(s) produces a deposition rate of 1 to 4 microns per hour. The target preferably is a cylinder or a plate.

Systems comprising embolization devices that have a significantly improved implantation behavior, and systems with devices as described are entirely visualized allowing improved delivery and retention of devices in the aneurysm or vesicle requiring occlusion. Systems generally include a medical device or coil having a surface, wherein the body portion has a radiopaque electropositive surface under physiological conditions when the device is implanted into an aneurysm or vesicle.

Systems may have at least an embolization device, wherein the body portion is configured to have a radiopaque and electropositive surface under physiological conditions when the device is implanted into an artery, aneurysm or vesicle.

Vascular implant systems comprise in combination an implant assembly including an elongate pushing member having a proximal end and a distal end, an implant coupled to the distal end of the elongate pushing member, and an electrolytically detachable zone proximate the distal end of the elongate pusher member, the electrolytically detachable zone comprising a stainless steel wire having a diameter at the electrolytically detachable zone of between at least about 0.457 mm (.0015") and about 0.762 mm (.0025") and an having an electrolytically detachable zone length of between at least about 6.096 mm (.002") and 24.384 mm (.008"), wherein the implant is configured to be electrolytically detachable from elongate pushing member at the electrolytically detachable zone; and an electrical power supply configured to electrically couple to the implant assembly at the proximal end of the elongate pushing member, the electrical power supply having a voltage of between about 11.5V and about 17.0V and configured to operate at a current between about 1.4 mA and about 2-4 mA. Systems maycomprise a 35N LT (high performance alloy) with implant history in clips.

Various objects, features, aspects and advantages of the inventive subject matter will become more apparent from the following detailed description of preferred embodiments, along with the accompanying drawing figures in which like numerals represent like components, to the extent feasible.

### BRIEF DESCRIPTION OF THE DRAWINGS

Fig. 1 is a perspective view of a medical device used with aneurysm embolization devices.
Fig. 2 is a top view of a Ta target medical device surrounding a medical device.
Fig. 3 is a side cross-sectional view of the target surrounding medical devices.
Fig. 4 illustrates a cross section of a conformal coating of Ta on a strut of a medical device.
Fig. 5 is an electron micrograph (cross section) of an exemplary embolization device with a tantalum layer according to the inventive subject matter.
Fig. 6 is an electron micrograph (top view) of an exemplary embolization device with a tantalum layer according to the inventive subject matter.
FIG. 7 is a side cross-sectional view of the target surrounding medical devices in position C of FIG. 3 with a plate above the medical devices.
FIG. 8 is a top view of a Ta target surrounding medical devices.
FIG. 9 is a side cross-sectional view of the target surrounding medical devices.
FIG. 10 is a side elevation view of medical devices positioned beside a planar target at a high angle of incidence.
FIG. 11 is a perspective view of a vasoocclusive implant according to another embodiment of the invention.
FIG. 12 is a perspective of a vasoocclusive implant according to another embodiment of the invention.
FIG. 13 is a circuit diagram of the electrical power supply coupled to an electrolytically detachable implant assembly that is inserted within a patient.

### DETAILED DESCRIPTION OF THE DISCLOSURE

The present inventors have assembled systems which are substantially more visible than prior art teachings, and have discovered that using MRI-safe devices (those which present no additional risks to patients) having higher nickel content and titanium allows for artifact free imaging.

Tantalum has a high atomic number and is also biomedically inert and corrosion resistant, making it an attractive material for radiopaque coatings in this application. It is kown that 3 to 10 microns of Ta is sufficiently thick to produce good X-ray contrast. However, because Ta has a melting point of almost 3000 C., any coating process must take place at a low homologous temperature (the ratio of the deposition temperature to the melting temperature of the coating material in degrees Kelvin) to preserve the A_{f} values of the medical devices as described previously. It is well known in the art of physical vapor deposition that low homologous coating temperatures often result in poor coating properties. Nevertheless, it has been unexpectedly found that radiopaque Ta coatings deposited under the correct conditions are able to withstand the strains inherent in stent use without unacceptable flaking.

The approach to deposit these adherent coatings at high rates with no direct control of the stent temperature without substantially affecting the temperature.

This disclosure relates to coatings that render biomedical devices including intraluminal biomedical devices radiopaque and that withstand the extremely high strains inherent in the use of such devices without unacceptable delamination. Specifically, it relates to coatings ofTahaving these properties and methods for applying them that do not adversely affect the thermomechanical properties of medical devices.

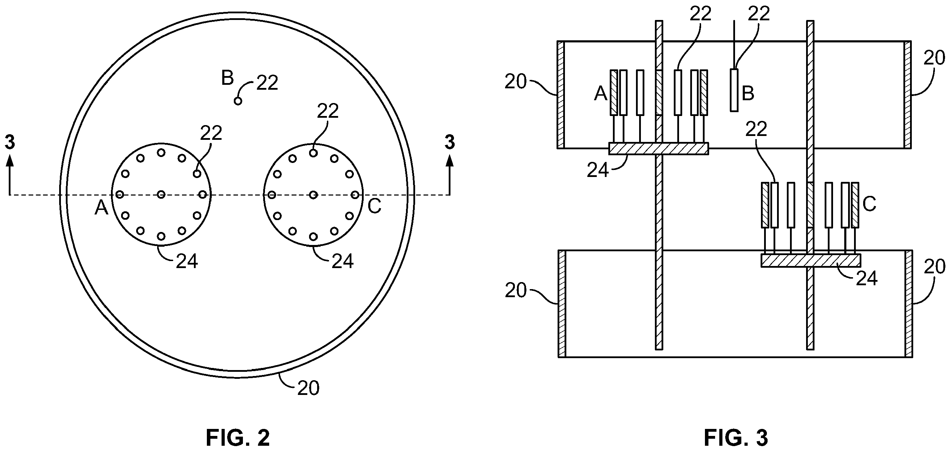

An unbalanced cylindrical magnetron sputtering system described in U.S. Pat. No. 6,497,803 B2, which is incorporated herein by reference, was used to deposit the coatings. FIGS. 2 and 3 illustrate the setup. Two Ta targets **20,** each 34 cm in diameter and 10 cm high, separated by 10 cm, were used. They were driven with either DC power or AC power at 40 kHz. Xenon or krypton was used as the sputter gas. The total power to both cathodes was either 2 kW or 4 kW and a bias of either -50 V or -150 V was applied to the medical devices during coating. Other devices well known to those in the art, such as vacuum pumps, power supplies, gas flow meters, pressure measuring equipment, are omitted from FIGS. 2 and 3 for clarity.

In each coating run, medical devices **22** were placed at one of three positions, as shown in FIGS. 2 and 3:

Position A-The stents were held on a 10 cm diameter fixture **24** that rotated about a vertical axis, which was approximately 7 cm from the cathode centerline. The vertical position of the medical devices was in the center of the upper cathode. Finally, each medical device was periodically rotated about its own vertical axis by a small "kicker", in a manner well known in the art.

Position B-The medical devices **22** were supported from a rotating axis that was approximately 7 cm from the chamber centerline. The vertical position of the stents was in the center of the upper cathode.

Position C-The medical devices **22** were on a 10 cm diameter fixture or plate **24** that rotated about a vertical axis, which was approximately 7 cm from the cathode centerline. The vertical position of the stents was in the center of the chamber, midway between the upper and lower cathodes. Finally, each medical device was periodically rotated about its own vertical axis with a "kicker."

Prior to coating, the medical devices were cleaned with a warm aqueous cleaner in an ultrasonic bath. Crest 270 Cleaner (Crest Ultrasonics, Inc.) diluted to 0.5 pounds per gallon of water was used at a temperature of 55 C. This ultrasonic detergent cleaning was done for 10 minutes. The medical devices were then rinsed for 2 minutes in ultrasonically agitated tap water and 2 minutes in ultrasonically agitated de-ionized water. The stents were then blown dry with nitrogen and further dried with hot air. The manner in which the stents were cleaned was found to be very important. When the stents were cleaned ultrasonically in acetone and isopropyl alcohol, a residue could be seen on the stents that resulted in poor adhesion. This residue may be a consequence of material left after the electropolishing process, which is often done using aqueous solutions.

The Ta sputtering targets were preconditioned at the power and pressure to be used in that particular coating run for 10 minutes. During this step a shutter isolated the medical devices from the targets. This preheating allowed the medical devices to further degas and approach the actual temperature of the coating step. After opening the shutter, the coating time was adjusted so that a coating thickness of approximately 10 microns resulted. At a power of 4 kW the time was 2 hours and 15 minutes and at a power of 2 kW the time was 4hours and 30 minutes. These are very acceptable coating rates for a manufacturing process. The medical devices were not heated or cooled directly in any way during deposition. Their time-temperature history was determined entirely by the coating process.

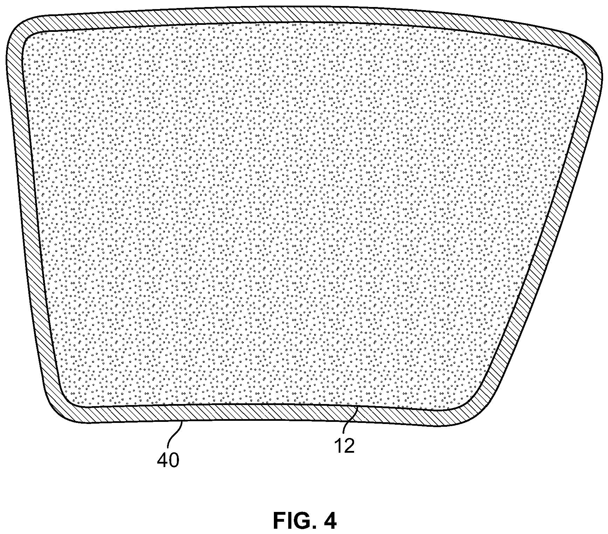

FIG. 4 illustrates the cross section of a conformal coating of Ta 40 on a strut 12, shown approximately to scale for a 10-micron thick coating. Medical devices coated in this manner were evaluated in several ways. First, they were pressed into adhesive tape to see if there was any flaking or removal when the tape was peeled away. Next, the medical devices were flexed to their maximum extent and examined for flaking. In all cases this flexing was done at least three times and in some cases it was done as many as ten times. Finally, the *At* values for the medical devices were measured by determining the temperature at which they recovered their original shape using a water bath.

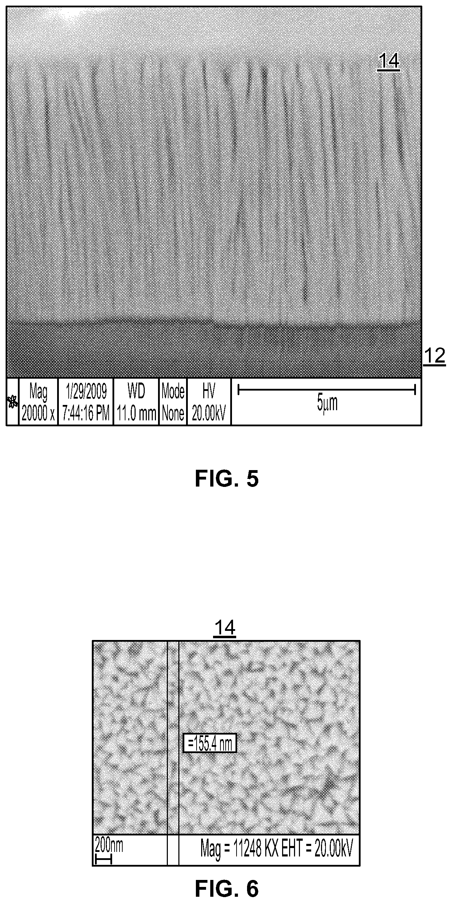

As charted in FIG. 5, the reflectance was measured to be about 0.5% at a wavelength of 400 nm and rises to about 1.10% at 700 nm. This is an emissivity of approximately 99% or greater across the visible spectrum.

The combination of a very low *A_{f}* and excellent adhesion is very surprising. Without being bound to this explanation, one possibility consistent with the observed results is that the coating is very porous. Low homologous temperatures (the ratio of the substrate temperature during coating to the melting point of the coating material, in degrees Kelvin) are known to produce open, columnar coating structures. The observed black appearance may be the result of an extremely porous coating. It is also known in the art that such morphology is also associated with very low coating stress, since the coating has less than full density. However, even if this explanation is correct, the excellent adhesion is very surprising. Typically such porous coatings have very poor adhesion and we were able to aggressively flex the coating with no indication of flaking.

Another possible consequence of the high emissivity of the coating is the fact that the radiative cooling of the medical device during coating is more effective, thereby helping to maintain a low coating temperature.

Furthermore, sputtered Ta typically exists in one of two crystalline phases, either tetragonal (known as the beta phase) or body centered cubic (known as the alpha phase). The alpha phase of Ta is much more ductile than the beta phase and can withstand greater strains. Therefore, the alpha phase of Ta is more desirable in this application.

There is also nothing in the prior art to suggest that alpha Ta can be deposited in such an open, porous structure.

An open, porous structure may have other advantages as well. For example, the microvoids in the coating would permit the incorporation of drugs or other materials that diff-use out over time. In the art, drug-eluting coatings on medical devices are presently made using polymeric materials. A porous inorganic coating would allow drug-eluting stents to be made without polymeric overcoats.

Surprisingly, the medical devices at position C all had adhesion equal to or better than the stents at positions A and B, regardless of conditions. Table 2 illustrates the surprising results. (NA indicates coating runs for which no data was taken at those positions.) The medical devices at position C always had very little or no flaking, even under coating conditions where medical devices in positions A or B had significant flaking.

Medical devices in position C receive a generally more oblique and lower energy coating flux than stents in positions A or B. By an oblique coating flux we mean that the majority of the depositing atoms arrive in directions that are not generally perpendicular to the surface being coated. Some of the atoms arriving at the surfaces of the medical devices in position C from the upper and lower targets will have done so without losing significant energy or directionality because of collisions with the background sputter gas. Those atoms, most of which will come from portions of the targets close to the medical devices as seen in FIGS. 2 and 3, will create an oblique coating flux. Other atoms will undergo several collisions with the background gas and lose energy and directionality before arriving at the substrate surfaces. Those atoms, which will generally come from portions of the targets at greater distances, will form a low energy coating flux. Westwood has calculated ("Calculation of deposition rates in diode sputtering systems," W. D. Westwood, Journal of Vacuum Science and Technology, Vol. 15 page 1 (1978)) that the average distance a Ta atom goes in Ar at 3.4 mTorr before its energy is reduced to that of the background gas is between about 15 and 30 cm. (The distance would be somewhat less in Kr and the exact value depends on the initial energy of the Ta atom.) Because our cylindrical targets have an inside diameter of approximately 34 cm, substrates placed in the planes of the targets (positions A and B) receive a greater number of high energy, normal incidence atoms than those placed between the targets (position C).

The geometry of the cylindrical magnetron arrangement shown in FIGS. 2 and 3 assures that atoms arriving at the surface of the medical devices in position C will do so either at relatively oblique angles or with relatively low energy. Referring to FIGS. 2 and 3, when the medical devices are close to the targets, where the arriving Ta atoms have lost little energy, the atoms arrive at oblique angles. When the medical devices move closer to the center of the chamber where the arrival angles are less oblique, they are farther from the target surface so that the arriving Ta atoms have lost energy through gas collisions.

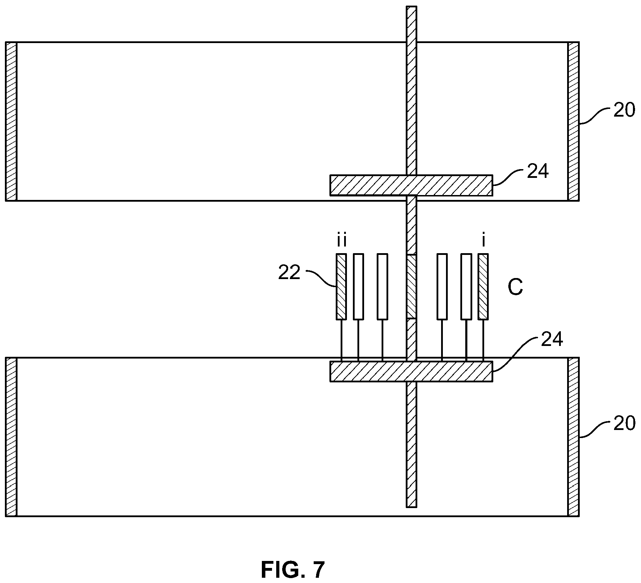

The non-uniformity in appearance that resulted with the fixturing shown in FIGS. 2 and 3 in position C indicates that the coating structure depends on the details of how the medical devices and sputter targets are positioned relative to one another. As discussed earlier, when the stents are in position C1 in FIG. 3, they receive very oblique incidence material from portions of the targets that are close, while the coating material that arrives from other portions of the target has to travel farther. Therefore, all of the coating flux has arrived at high angles or has traveled a considerable distance and has lost energy and directionality through collisions with the sputtering gas. When the medical devices are in position C2 in FIG. 3, however, they receive a somewhat less oblique coating from all directions. In the configuration shown in FIG. 3, position C the bottoms of the medical devices are shielded from the more direct flux from the bottom target by the plate that holds them, but the tops of the medical devices are not similarly shielded from the more direct flux coming from the top target. By adding the plate above the stents shown in FIG. 7, the more direct coating flux is shielded at all points on the medical devices and the coating material either arrives at relatively oblique incidence or after scattering from the background gas and losing energy and directionality. The plate above the medical devices restores the symmetry of the situation and the coatings on the stents become uniformly black overall.

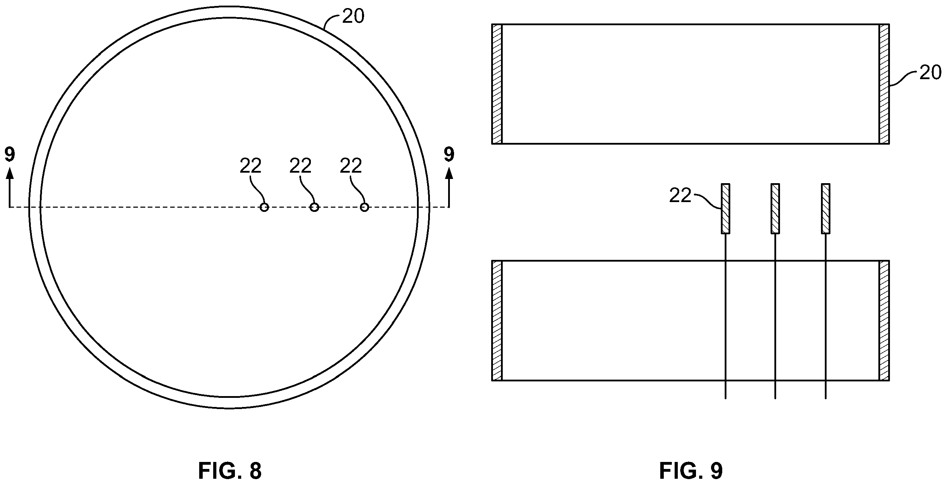

Other methods of positioning and moving the substrates within the chamber can also produce results similar to those described above and are within the scope of the invention. In another experiment three medical devices were located as shown in FIGS. 8 and 9. All three medical devices 22 were held fixed at their positions within the chamber and were rotated about their individual vertical axes during the coating run. The innermost stent was 3 cm from the cathode centerline, the middle stent was 7 cm from the cathode centerline and the outermost stent was 11 cm from the cathode centerline. The deposition was done at a DC power of 2 kW, a Kr pressure of 3.4 mTorr and with the stents biased at 50 V.

An alternative, although less desirable, approach to oblique incidence coatings or large target to substrate distances in order to reduce the energy of the arriving atoms through collisions is to raise the pressure of the sputtering gas.

Sputtering talkes place under conditions of continuous gas flow. That is, the sputtering gas is brought into the chamber at a constant rate and is removed from the chamber at the same rate, resulting in a fixed pressure and continuous purging of the gas in the chamber. This flow is needed to remove unwanted gases, such as water vapor, that evolve from the system during coating. These unwanted gases can become incorporated in the growing coating and affect its properties.

The high vacuum pumps used in sputtering, such as diffusion pumps, turbomolecular pumps and cryogenic pumps, are limited with respect to the pressure that they can tolerate at their openings. Therefore, it is well known that in order to achieve high sputtering pressures it is necessary to "throttle" such pumps, or place a restriction in the pump opening that permits the chamber pressure to be significantly higher than the pressure at the pump. Such "throttling" necessarily reduces the flow of gas through the chamber, or gas throughput. Surprisingly, we have found that the adherence of the coatings is improved at high gas throughputs.

In another experiment, coatings were done on medical devices in the C position using the 34 cm diameter dual cathode shown in FIGS. 2 and 3. The sputtering gas was Kr at a pressure of 0.453 Pa (3.4 mTorr). A DC power of 2 kW was used together with a substrate bias of - 50 V, the conditions of Run No. 8 in Table 2. The Kr flow was 28 standard cubic centimeters per minute, or 0.36 Torr-liters per second. At a pressure of 0.453 Pa (3.4 mTorr), this corresponds to a throttled pumping speed of 104 liters per second during the process. The resulting black coatings all flaked at levels between level 1 and level 3 when tested. The position of the pump throttle was then changed and the Kr flow was increased to 200 standard cubic centimeters per minute 2.53 Torr-liters per second. Coatings were done on medical devices in the C position at the same power, pressure and bias levels as before. The only difference was that the throttled pumping speed during this process was 744 liters per second. In this case, there were no flakes or cracks in the coating evident after testing.

Based on the foregoing results, we conclude that adequate adhesion does not result at low gas throughputs, which are usually necessary to achieve high sputtering pressures. The sputtering pressure and system geometry must be chosen together so that the coating flux arrives at the substrate surface either at high angles of incidence or after the sputtered atoms have traveled a sufficient distance from the target to reduce their energies significantly.

While the geometry of a cylindrical magnetron makes this possible in an efficient way, as we have shown, the same results can be accomplished using planar targets as well. In the case of planar targets, the requirement is to place the substrates far enough from the target surface(s) that a large target-to-substrate distance is achieved. Alternatively, the substrates could be placed to the side of a planar target so that the material arrives at high incidence angles.

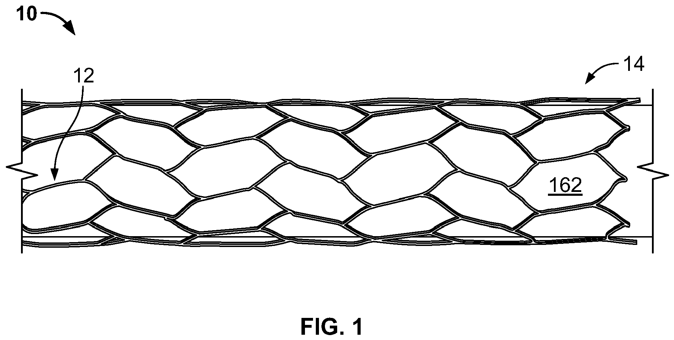

Fig. 1 depicts an embolization device 10 having a surface 12 that is coated with a tantalum coating 14. The coating 14, is coupled to the surface of embolization device 12, cell 162 is defined by geometric shape desired and has a coated surface. In further detail, and still referring to Fig. 1, the embolization device surface 12 has sufficient width and surface area to apply a tantalum coating, when device diameter is, for example, from at least 2.0 to 5.0 mm and length is 1cm to 5cm. The tantalum coating 14 is radiopaque and positively charged to be visualized the entire length and attract and ionically interacts with negatively charged bodily fluids such as cells, blood, elements such as oxygen or tissue to the embolization device surface 12. creating a stable placement to maintain embolization device location and position.

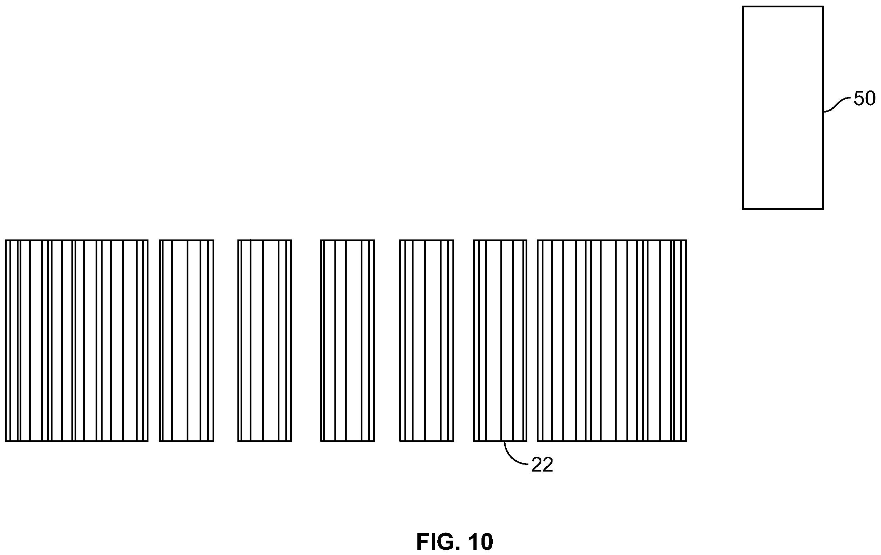

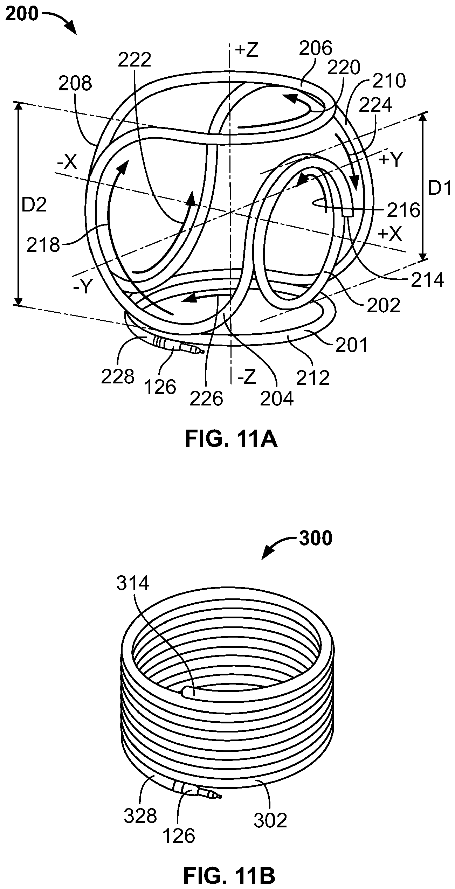

FIGS. 11A and 11B illustrate vacoocclusive implants according to embodiments of the invention. FIG. 11.A illustrates a framing microcoil implant 200 made from an embolic coil 201, and having a box shape which approximates a spheroid when placed within an aneurysm. Loops 202, 204, 206, 208, 210, 212 are wound on three axes: an X-axis extending in the negative direction (-X) and a positive direction (+X) from a coordinate origin (0), a Y-axis extending in the negative direction (-Y) and a positive direction (+Y) from the coordinate origin (0), and an Z-axis extending in the negative direction (-Z) and a positive direction (+Z) from the coordinate origin (0). A first loop 202 having a diameter Di begins at a first end 214 of the embolic coil 201 and extends around the +X-axis in a direction 216. As depicted in FIG. 10, the first loop 202 includes approximately 1 Vi revolutions, but may (along with the other loops 204, 206, 208, 210, 2012) include between Vi revolution and 10 revolutions. The second loop 204 having a diameter D2 continues from loop 202 and extends around the-Y-axis in a direction 218. The third loop 206 then extends around the +Z-axis in a direction 220. The fourth loop 208 then extends around the -X-axis in a direction 222 The fifth loop 210 then extends around the +Y-axis in a direction 224. And finally, the sixth loop 212 extends around the Z-axis in a direction 226. As seen in FIG. 11A, subsequent to the forming of the loops 202, 204, 206, 208, 210, 212, the coupling joint 126 is formed at a second end 228 of the embolic coil 201.

This framing microcoil implant 200 is configured for being the initial microcoil placed within an aneurysm, and therefore, in this embodiment, loops 204, 206, 208, 210 and 212 all have a diameter approximately equal to D2. The first loop 202,
however, is configured to be the first loop introduced into the artery, and in order to maximize the ability of the microcoil implant 200 to stay within the aneurysm during coiling, the diameter Di of the first loop 202 is to between 65% and 75% of the diameter D2, and more particularly, about 70% of the diameter of D2. Assuming that D2 is chosen to approximate the diameter of the aneurysm, when the first loop 202 of the microcoil implant 200 is inserted within the aneurysm, as it makes its way circumferentially around the wall of the aneurysm, it will undershoot the diameter of the aneurysm if and when it passes over the opening at the aneurysm neck, and thus will remain within the confined of the aneurysm. Upon assembly of the microcoil implant 200 into the vascoocclusive implant system 100, the choice of the tether 132 can be important for creating a microcoil implant 200 that behaves well as a framing microcoil, framing the aneurysm and creating a supportive lattice to aid subsequent coiling, both packing and finishing.

For example, the tether 132 may be made from 0.274 mm (.0009") diameter PET thread in microcoil implants 200 having a diameter D2 of 5 mm or less, while the tether 132 may be made from 0.671 mm (.0022") diameter Engage® brand of thread in microcoil implants 200 having a diameter D2 of 5 mm or more. In addition, the diameter of the wire 144, if 92/8 Pt/W, may be chosen as 0.457 mm (.0015") in 33.528 mm (.0.11") diameter embolic coils 130 and 0.610 mm (.002") in 3.658 mm (.012") diameter embolic coils 130. The 33.53 mm (.011") embolic coils 130 may be chosen for the construction of microcoil implants 200 having a diameter D2 of 4.5 mm or less, and the 3.658 mm (.012") diameter embolic coils 130 may be chosen for the construction of microcoil implants 200 having a diameter D2 of 4.5 mm or more.

In microcoil implants 200 having a diameter D2 or 6 mm or larger, additional framing microcoil models may be made having 30962 mm (0.013") or larger embolic coils 130 wound with 0.610 mm (.002") and larger wire 144. It should be noted that the coiling procedure need not necessarily use only one framing microcoil, and that during the implantation procedure, one or more framing microcoils may be used to set up the aneurysm for filling microcoils and finishing microcoils.

The wire 144 used in making the embolic coil 130 used to construct the filling microcoil implant 300 may be 92/8 Pt/W wire of a diameter between about 0.533 mm (.00175") and 0.838 mm (.00275"), and more particularly between 0.610 mm (.002") and 0.686 mm (.00225"). The outer diameter of the embolic coil 130 of the filling microcoil implant 300 may be between 3.353 mm (.011") and 3.962 mm (.013"), more particularly about 3.658 mm (.012"). One or more filling microcoil implants 300 can be used after one or more framing coil implants 200 have been placed in the aneurysm, to pack and fill as much volume of the aneurysm as possible. The comparatively soft nature of the filling microcoil implants 300 allows a sufficient amount of packing to achieve good thrombosis and occlusion, without creating potentially dangerous stresses on the wall of the aneurysm that could potentially lead to rupture (or re-rupture). In addition to the use of a helically shaped microcoil as a filling microcoil implant 300, they may also be used as a finishing microcoil implant, which is the last one or more implant that are placed at the neck of the aneurysm to engage well with the coil mass while maximizing the filled volume at the neck of the aneurysm. These finishing microcoils are typically smaller, having an outer diameter of about 3.048 mm (.010"), and being wound from 92/8 Pt/W wire having a diameter of between 3.048 mm (.001 ") to 0.479 mm (.00175"), more particularly between 0.381 mm (.00125") and (.0015"). The tether 132 used in a helical finishing microcoil may comprise 0.305 mm (.001") PET thread.

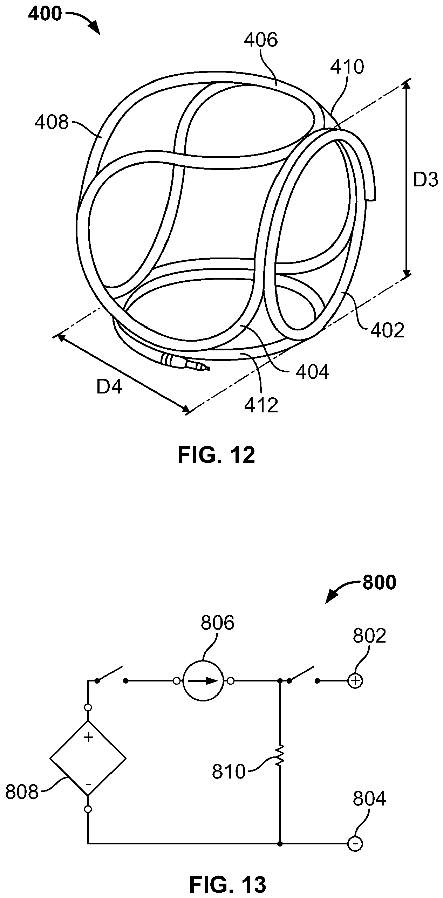

FIG. 12 illustrates a complex microcoil implant 400, having a first loop 402, second loop 404, third loop 406, fourth loop 408, fifth loop 410, and sixth loop 412, wound in three axes, much like the microcoil implant 200 of FIG. 10. However, the Diameter D3 of the first loop 402 is about the same as the diameter D4 of each of the other loops 404, 406, 408, 410, 412. Therefore the mandrel 500 used in the construction of the loops 402, 404, 406, 408, 410, 412 would include a first arm 502 having a similar diameter to the other arms 504, 506, 508, 510, 512. A complex microcoil implant 400 of this construction may be used as a framing microcoil implant, but may alternatively by used as a finishing microcoil implant. The complex or three-dimensional structure in many clinical situations can aid in better engagement of the finishing microcoil implant with the rest of the coil mass, due to its ability to interlock. There is thus less chance of the finishing microcoil implant migrating out of the aneurysm, into the parent artery.

Placement of a first "framing" microcoil within an aneurysm is often done using a three-dimensional, or "complex" microcoil (a microcoil which is wound around a plurality of axes). The initial framing microcoil is the base structure into which later "filling" microcoils are packed. As the first microcoil placed into a completely uncoiled aneurysm, even if it is a three-dimensional or complex microcoil, the first loop of the microcoil may exit from the aneurysm after it has entered, instead of looping several times around the inside of the aneurysm. This is exacerbated by the absence of a prior microcoil, whose structure tends to help subsequently placed coils stay within the aneurysm. Microcoils in which all loops are formed at substantially the same diameter are especially prone to this exiting phenomenon when used as the first framing microcoil.

Referring now to Figure 13, those skilled in the art understand that detachment between at least about 4 and 14 seconds, are achieved by actuating the instant system. Clinically, consistent detachment times under 10 seconds have been achieved. (Blockade Medical, LLC, Irvine, California 92693).

Moreover, as used herein, and unless the context dictates otherwise, the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

In one aspect of the system of the inventive subject matter, an embolization device has a body portion made from a metal or metal alloy known in the art of stent assisted coil embolization. For example, contemplated body portions may comprise stainless steel, titanium, nitinol, etc. The medical device diameter is typically between 2.0 and 5.0 mm, the medical device wall thickness is typically between 30 and 100 microns and the stent surface is coated via vapor deposition with a thin 5 to 30 micron layer of tantalum. Likewise, 35N LT (high performance alloy) is used for those aspects of the delivery system and implant needed, to avoid MRI artifacts.

Likewise, 35N LT (high performance alloy - not stainless steel) which has higher nickel content than 304316 and Titanium - which has been used in aneurysm clips, has not been used with detachable delivery systems, but is effective to use with the instant teachings.

The advantages of the present inventive subject matter include, without limitation, that the devices contemplated herein are highly visible/radiopaque and able to attract negatively charged bodily materials to create to stable embolization of an aneurysm sac or vesicle, via direct binding, coagulation, or indirect binding. Such attraction/binding to fluids and their components, cells, tissue, proteins, is thought to be readily achieved because most of such materials are more electronegative (relative to tantalum or other electropositive materials) while tantalum is positively charged/electropositive. Further, devices according to the inventive subject matter will generally pass through most common and commercially available microcatheters without any deliverability concerns or issues as otherwise associated with standard braided stents or laser-cut tube stents. Further, the devices can easily be repositioned within the artery, aneurysm or vesicle if first placement is undesirable.

It should be apparent to those skilled in the art that many more modifications besides those already described are possible without departing from the inventive concepts herein. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

To the extent that insubstantial substitutes are made, to the extent that the applicant did not in fact draft any claim so as to literally encompass any particular embodiment, and to the extent otherwise applicable, the applicant should not be understood to have in any way intended to or actually relinquished such coverage as the applicant simply may not have been able to anticipate all eventualities; one skilled in the art, should not be reasonably expected to have drafted a claim that would have literally encompassed such alternative embodiments.

Further, the use of the transitional phrase "comprising" is used to maintain the "open-end" claims herein, according to traditional claim interpretation. Thus, unless the context requires otherwise, it should be understood that the term "comprise" or variations such as "comprises" or "comprising", are intended to imply the inclusion of a stated element or step or group of elements or steps but not the exclusion of any other element or step or group of elements or steps.

Such terms should be interpreted in their most expansive forms so as to afford the applicant the broadest coverage legally permissible.

## Claims

1. An intraluminal medical device system for placement within a neurovascular structure, which comprises, in combination:
at least a flexible body; and
a porous columnar metallic coating comprising a plurality of independent columnar structures separated from one another by an opening and disposed on at least a portion of the flexible body, wherein
the porous columnar metallic coating extends in a substantially normal direction from a surface of at least a portion of the flexible body;
flaking of the porous columnar metallic coating being less than 1% of the porous columnar metallic coated surface when the flexible body and porous columnar metallic coating are strained up to 8%; and
the combination of the size of the columnar structures of the porous columnar metallic coating and the size of the openings between the columnar structures of the porous columnar metallic coating results in an emissivity in the visible spectrum range of at least 80%;
wherein the flexible body partially comprises a radio transparent material and the porous columnar metallic coating on at least a portion of the flexible body comprises tantalum; wherein the porous columnar tantalum coating thickness is between approximately 3 and 10 microns;
wherein the porous columnar tantalum coating is applied to the flexible body by a physical vapor deposition process;
wherein the physical vapor deposition process includes one of the group of sputtering, cathodic arc deposition or thermal evaporation; and
the intraluminal medical device is an embolic coil;
further comprising a material in the porous columnar tantalum coating, wherein the material diffuses out overtime.

2. The intraluminal medical device of claim 1, wherein the flexible body further comprises:
an elastic shape retaining body having an outer surface, namely an embolic coil; the porous columnar metallic coating is disposed on at least a portion of the outer surface;
wherein the porous columnar metallic coating is applied using a physical vapor deposition process.

3. The intraluminal medical device of claim 1, wherein the flexible body further comprises an elastic shape retaining body at least partially comprising a material having superelasticity and shape memory.

4. The intraluminal medical device of claim 3, wherein the porous columnar metallic coating is a radiopaque coating.

5. The intraluminal medical device of claim 3, wherein the flexible body comprises at least one of nitinol, and other high performance alloys, including 35N LT.

6. The intraluminal medical device of any preceding claim, wherein the flexible body comprises 35N LT.

7. The intraluminal medical device of claim 1, wherein the flexible body is fabricated from a shape memory alloy having an A_{f} value below body temperature.

8. The intraluminal medical device of any preceding claim, wherein the medical device diameter is between 2.0 and 5.0 mm.

9. The intraluminal medical device of any preceding claim, wherein the medical device length is between 1 and 5 cm.

10. The intraluminal medical device of any preceding claim, wherein the medical device thickness is between 30 and 100 microns.

11. A process for depositing a tantalum layer on the medical device of any preceding claim, the method comprising the steps of:
maintaining a background pressure of inert gas in a sputter coating system containing a tantalum sputter target;
applying a voltage to the tantalum target to cause sputtering; and
sputtering for a period of time to produce the desired coating thickness;
wherein the tantalum layer preferably has an emissivity in the visible spectrum of at least 80%.

## Patentansprüche

1. Intraluminales medizinisches Vorrichtungssystem zur Platzierung innerhalb einer neurovaskulären Struktur, das in Kombination Folgendes umfasst:
mindestens einen flexiblen Körper; und
eine poröse säulenförmige Metallbeschichtung, die mehrere unabhängige säulenförmige Strukturen umfasst, die durch eine Öffnung voneinander getrennt und auf mindestens einem Abschnitt des flexiblen Körpers angeordnet sind, wobei sich die poröse säulenförmige Metallbeschichtung in einer im Wesentlichen senkrechten Richtung von einer Oberfläche von mindestens einem Abschnitt des flexiblen Körpers erstreckt;
Abschuppen der porösen säulenförmigen Metallbeschichtung, die weniger als 1 % der porösen säulenförmigen Metallbeschichtungsoberfläche ausmacht, wenn der flexible Körper und die poröse säulenförmige Metallbeschichtung bis zu 8 % verspannt werden; und
wobei die Kombination der Größe der säulenförmigen Strukturen der porösen säulenförmigen Metallbeschichtung und der Größe der Öffnungen zwischen den säulenförmigen Strukturen der porösen säulenförmigen Metallbeschichtung zu einem Emissionsvermögen im sichtbaren Spektralbereich von mindestens 80 % führt;
wobei der flexible Körper teilweise ein strahlentransparentes Material aufweist und die poröse säulenförmige Metallbeschichtung auf mindestens einem Abschnitt des flexiblen Körpers Tantal aufweist;
wobei die Dicke der porösen säulenförmigen Tantalbeschichtung zwischen ungefähr 3 und 10 Mikron liegt;
wobei die poröse säulenförmige Tantalbeschichtung durch einen physikalisches Dampfabscheidungsprozess auf den flexiblen Körper aufgetragen wird;
wobei der physikalische Dampfabscheidungsprozess eines aus der Gruppen bestehend aus Sputtern, kathodischer Lichtbogenabscheidung oder thermischer Verdampfung umfasst; und
die intraluminale medizinische Vorrichtung eine Emboliespirale ist;
ferner umfassend ein Material in der porösen säulenförmigen Tantalbeschichtung, wobei das Material mit der Zeit ausdiffundiert.

2. Intraluminale medizinische Vorrichtung nach Anspruch 1, wobei der flexible Körper ferner Folgendes umfasst:
einen elastischen Formhaltekörper mit einer Außenfläche, nämlich einer Emboliespirale; wobei die poröse säulenförmige Metallbeschichtung auf mindestens einem Abschnitt der Außenfläche angeordnet ist;
wobei die poröse säulenförmige Metallbeschichtung unter Verwendung eines physikalischen Dampfabscheidungsprozesses aufgebracht wird.

3. Intraluminale medizinische Vorrichtung nach Anspruch 1, wobei der flexible Körper ferner einen elastischen Formhaltekörper umfasst, der mindestens teilweise ein Material mit Superelastizität und Formgedächtnis umfasst.

4. Intraluminale medizinische Vorrichtung nach Anspruch 3, wobei die poröse säulenförmige Metallbeschichtung eine röntgendichte Beschichtung ist.

5. Intraluminale medizinische Vorrichtung nach Anspruch 3, wobei der flexible Körper mindestens eines von Nitinol und anderen Hochleistungslegierungen, einschließlich 35N LT, umfasst.

6. Intraluminale medizinische Vorrichtung nach einem der vorhergehenden Ansprüche, wobei der flexible Körper 35N LT umfasst.

7. Intraluminale medizinische Vorrichtung nach Anspruch 1, wobei der flexible Körper aus einer Formgedächtnislegierung mit einem A_{f} -Wert unterhalb der Körpertemperatur hergestellt ist.

8. Intraluminale medizinische Vorrichtung nach einem der vorhergehenden Ansprüche, wobei der Durchmesser der medizinischen Vorrichtung zwischen 2,0 und 5,0 mm liegt.

9. Intraluminale medizinische Vorrichtung nach einem der vorhergehenden Ansprüche, wobei die Länge der medizinischen Vorrichtung zwischen 1 und 5 cm liegt.

10. Intraluminale medizinische Vorrichtung nach einem der vorhergehenden Ansprüche, wobei die Dicke der medizinischen Vorrichtung zwischen 30 und 100 Mikron liegt.

11. Verfahren zum Auftragen einer Tantalschicht auf der medizinischen Vorrichtung nach einem vorhergehenden Anspruch, wobei das Verfahren die folgenden Schritte umfasst:
Aufrechterhalten eines Inertgas-Hintergrunddrucks in einem Sputter-Beschichtungssystem, das ein Tantal-Sputtertarget enthält;
Anlegen einer Spannung an das Tantal-Target, um ein Sputtern zu verursachen; und
Sputtern über einen bestimmten Zeitraum, um die gewünschte Beschichtungsdicke zu erzeugen;
wobei die Tantalschicht vorzugsweise ein Emissionsvermögen im sichtbaren Spektrum von mindestens 80 % aufweist.

## Revendications

1. Système de dispositif médical intraluminal destiné à être placé à l'intérieur d'une structure neurovasculaire, qui comprend, en combinaison :
au moins un corps souple ; et
un revêtement métallique colonnaire poreux comprenant une pluralité de structures colonnaires indépendantes séparées les unes des autres par une ouverture et disposées sur au moins une partie du corps souple, dans lequel le revêtement métallique colonnaire poreux s'étend dans une direction sensiblement normale à partir d'une surface d'au moins une partie du corps souple ;
l'écaillage du revêtement métallique colonnaire poreux représentant moins de 1 % de la surface métallique colonnaire poreuse revêtue lorsque le corps souple et le revêtement métallique colonnaire poreux sont contraints jusqu'à 8 % ; et
la combinaison de la taille des structures colonnaires du revêtement métallique colonnaire poreux et de la taille des ouvertures entre les structures colonnaires du revêtement métallique colonnaire poreux se traduit par une émissivité dans la plage du spectre visible d'au moins 80 % ;
dans lequel le corps souple comprend partiellement un matériau radio-transparent et le revêtement métallique colonnaire poreux sur au moins une partie du corps souple comprend du tantale ;
dans lequel l'épaisseur du revêtement de tantale colonnaire poreux est comprise entre environ 3 et 10 microns ;
dans lequel le revêtement de tantale colonnaire poreux est appliqué sur le corps souple par un processus de dépôt physique en phase vapeur ;
dans lequel le processus de dépôt physique en phase vapeur comprend l'un parmi le groupe de pulvérisation cathodique, de dépôt à l'arc cathodique ou d'évaporation thermique ; et
le dispositif médical intraluminal est une spirale embolique ;
comprenant en outre un matériau dans le revêtement de tantale colonnaire poreux, le matériau se diffusant au fil du temps.

2. Dispositif médical intraluminal selon la revendication 1, dans lequel le corps souple comprend en outre :
un corps à maintien de forme élastique présentant une surface extérieure, à savoir une spirale embolique ; le revêtement métallique colonnaire poreux est disposé sur au moins une partie de la surface extérieure ;
dans lequel le revêtement métallique colonnaire poreux est appliqué à l'aide d'un processus de dépôt physique en phase vapeur.

3. Dispositif médical intraluminal selon la revendication 1, dans lequel le corps souple comprend en outre un corps à maintien de forme élastique comprenant au moins partiellement un matériau présentant une superélasticité et une mémoire de forme.

4. Dispositif médical intraluminal selon la revendication 3, dans lequel le revêtement métallique colonnaire poreux est un revêtement radio-opaque.

5. Dispositif médical intraluminal selon la revendication 3, dans lequel le corps souple comprend au moins l'un parmi le nitinol et d'autres alliages à haute performance, dont le 35N LT.

6. Dispositif médical intraluminal selon l'une quelconque des revendications précédentes, dans lequel le corps souple comprend du 35N LT.

7. Dispositif médical intraluminal selon la revendication 1, dans lequel le corps souple est fabriqué à partir d'un alliage à mémoire de forme présentant une valeur Af inférieure à la température corporelle.

8. Dispositif médical intraluminal selon l'une quelconque des revendications précédentes, dans lequel le diamètre du dispositif médical est compris entre 2,0 et 5,0 mm.

9. Dispositif médical intraluminal selon l'une quelconque des revendications précédentes, dans lequel la longueur du dispositif médical est comprise entre 1 et 5 cm.

10. Dispositif médical intraluminal selon l'une quelconque des revendications précédentes, dans lequel l'épaisseur du dispositif médical est comprise entre 30 et 100 microns.

11. Processus destiné au dépôt d'une couche de tantale sur le dispositif médical selon l'une quelconque des revendications précédentes, le procédé comprenant les étapes suivantes :
le maintien d'une pression de fond de gaz inerte dans un système de revêtement de pulvérisation contenant une cible de pulvérisation de tantale ;
l'application d'une tension à la cible de tantale pour provoquer une pulvérisation cathodique ; et
la pulvérisation pendant une période donnée pour produire l'épaisseur de revêtement souhaitée ;
dans lequel la couche de tantale présente de préférence une émissivité dans le spectre visible d'au moins 80 %.
